# EUROPEAN PATENT SPECIFICATION

(11) **EP 3 082 659 B1**
(45) Date of publication and mention of the grant of the patent: **22.05.2019**
(21) Application number: 14871272.2
(22) Date of filing: 08.12.2014
(51) Int. Cl.: A61F 2/64, A61F 5/01, A61F 2/50, A61F 2/68

(54) **ARTIFICIAL KNEE JOINT**
KÜNSTLICHES KNIEGELENK
ARTICULATION ARTIFICIELLE DE GENOU

(30) Priority: 19.12.2013 SE 1351537
(43) Date of publication of application: 26.10.2016
(73) Proprietor: Fillauer Europe AB, 191 27 Sollentuna (SE)
(72) Inventor: RAMIREZ, Christoffer, S-181 50 Vallentuna (SE)
(74) Representative: Brann AB
(86) International application number: PCT/SE2014/051467
(87) International publication number: WO 2015/094087

(56) References cited:
- FR-A1- 2 344 271
- US-A- 3 901 223
- US-A- 5 201 776
- US-A1- 2002 026 246

## Description

### TECHNICAL FIELD

The present invention relates to an artificial knee joint provided as a mechanical joint construction adapted for stabilising a lower extremity and supporting a normal walking step of the user as set out in claim 1.

### BACKGROUND

Walking is a complicated process and to be able to work efficiently, the lower extremities have to be able to 1: carry the body weight during the supporting phase, 2: rotate and coordinate the joints to provide a progression in the walking direction, 3: adjust the length of the extremity by means of bending the knee joint during the swing-phase, and 4: further decrease the movement of the body weight in height direction by means of bending the knee joint slightly during *midstance* (phase in the middle of the step). Lack of any of these functions can adventure a person's ability to walk.

Nevertheless, hinder of movement can be lessened by means of conventional orthosis-support by means of for instance a KAFO (Knee Ankle Foot Orthosis). A KAFO has to, to be energy-efficient, give full stability in the supporting phase and a natural and non-hindered swing-phase, as well as admit some bending of the knee joint in the middle of the step without adventuring the knee stability. An artificial knee joint also has to be designed such that it follows the natural poly-centric/multi-shaft pattern of movement of the knee.

For stabilisation of the human knee joint of individuals having reduced muscle strength and/or reduced joint control, to a large extent, knee joints having a locking function are used for providing the user stability during walking. These joints are only unlocked to provide a sitting position with bent knee. This locking has the disadvantage that the users will have an unnatural pattern of walking being energy-inefficient and in the long-run will give arise to wear damages.

Artificial knee joints by which the knee joint can be unlocked when initiating the swing phase are known within the technical field. US patent US 5490831, US 2943622 and the European patent application EP0872224 disclose knee joints using a pressure against the heel to provide a locking signal. According to these publications, a detector element is provided in the heel, which detecting element is connected to a locking element, for instance by means of a cable or rod. As long as the detector element detects a pressure against the heel, the joint is locked by means of the locking element. As long as there is no pressure against the heel, the knee joint is then released.

A significant disadvantage with the locking according to said document is that the knee joint also will be released if pressure is exerted against the frontal part of the user's foot. If the user tries to overcome a hindrance, such as a kerb, using his leg, the detector element will not detect any pressure against the heel and will hence not release the articulated joint. The user's knee joint will hence fold. As a result of this limitation the solution is unpractical and also dangerous.

Patent US 4632096 describes a joint that can be released by means of the foot being twisted relatively the leg. A cable is attached to the foot, which cable is connected to a locking element in the knee joint. By twisting the foot the locking of the joint can be released by means of the cable.

The disadvantage with the solution according to this US patent is that the release only can be performed by means of actively twisting the foot. This implies that the user has to be ready to twist his foot in each step. Furthermore, according to this solution, is also required a link between the knee joint and the foot or foot joint. This implies the functionality of the system depending on the structure of the surface of the ground.

Within the prosthesis technology, wherein user has no knee joint at all following an amputation, there are the so-called four-shaft knee joints for providing stability in the design of the knee joint. One example is given in patent US 5181931. Typical for these solutions is that the technology requires that the user does not have any own lower leg, and these solutions are thus disqualified as aiding means for many of the users of which the present invention is related to.

### SUMMARY OF THE INVENTION

FR 2 344 271 A1 discloses an artificial knee joint for artificial limb having two links joining upper and lower parts together and third link on slide to adjust pivoting axis. The knee joint is fitted to an artificial limb and has one pair of links providing the pivoting action. A third link has one end attached to a movable slide and enables the axis of pivoting to be adjusted to suit the user. The first set of links join together the upper and lower parts of the artificial knee joint by pivots these being on the outside. The third link is between the first two and has its upper pivot behind them. Its lower end pivots in the slide adjustable in a slot and locked by a screw.

The present invention solves the problems with prior art by means of the features of the appended claims. The invention also provides a solution that does not require any external mechanical control by means of wire connections or electronic control system to provide stability in the supporting phase. The geometrical stability of the knee joint according to the invention is also by its spring-tensioned sliding shaft adjustable for a wide range of weight classes.

According to a preferred embodiment of the present invention there is provided an artificial knee joint, as set out in claim 1.

Few parts and simple mechanics render the invention unique in its simplicity and reliability, wherein other solutions strive to be complex and hence too sensitive.

The polycentric movement of the joint of the present invention imitates the movement of the anatomic knee in a natural way such that the user is not limited in his range of movement.

### BRIEF DESCRIPTION OF DRAWINGS

Embodiments of the present invention will be explained in more detail with reference to appended drawing figures, wherein:
Figure 1 illustrates a view of a knee joint according to the invention incorporated in an artificial knee-ankle-foot orthosis (KAFO),
Figure 2 illustrates the knee joint in mounted position,
Figure 3 illustrates the specific components of the knee joint,
Figure 4 illustrates a geometric model of the knee joint,
Figure 5 illustrates the position of the knee joint over the thibio-femural joint, in sagittal view, and
Figure 6 - 14 illustrate the function of the joint seen in relation to the different phases of the walking cycle.

### DETAILED DESCRIPTION OF PREFERRED EMBODIMENTS

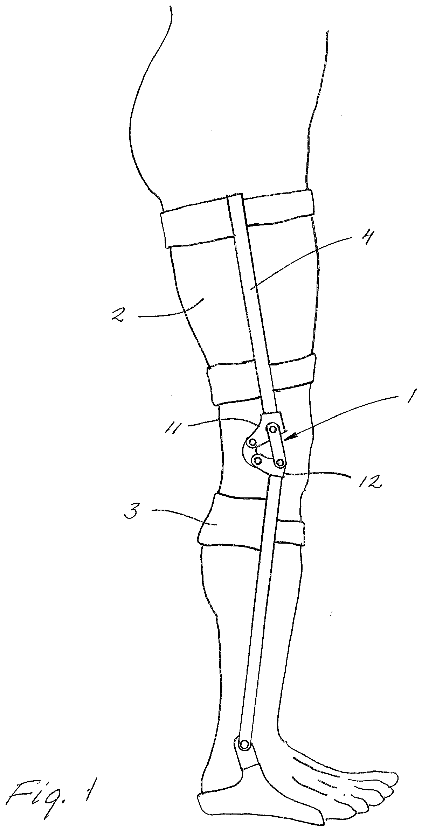

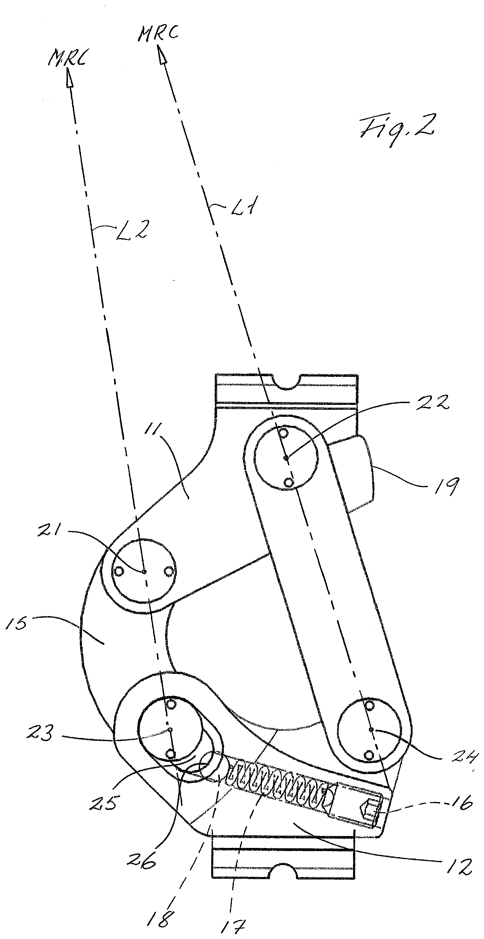

Figure 1 illustrates the invention embodied as an artificial knee joint 1 being incorporated in an orthopaedic means of assistance. The knee joint 1 comprise a first part 11 being rotatably connected to a second part 12 by means of links 13 and 14 as well as the link 15, such as illustrated in Figure 2.

The knee joint 1 is attachable to a user's leg 2. The first part 11 is adapted to be connected to a separately manufactured segment 4 attachable to the thigh of the wearer. Part 12 is in a similar way connectable to another separately manufactured segment 4 being attachable to the lower leg of the wearer.

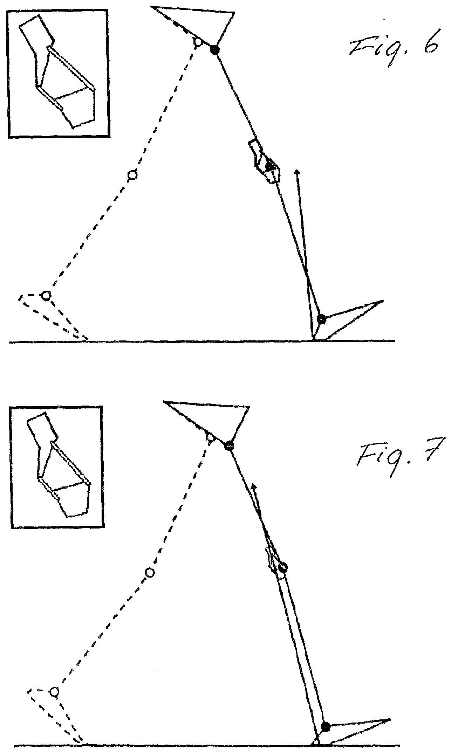

Figure 2 illustrates the knee joint in its initial position and can, depending on the (power)torques acting through the joint, either be released for rotation or locked by means of the links part 15 and 13-14 being positioned in parallel. This is further explained below with reference to Figures 6-15.

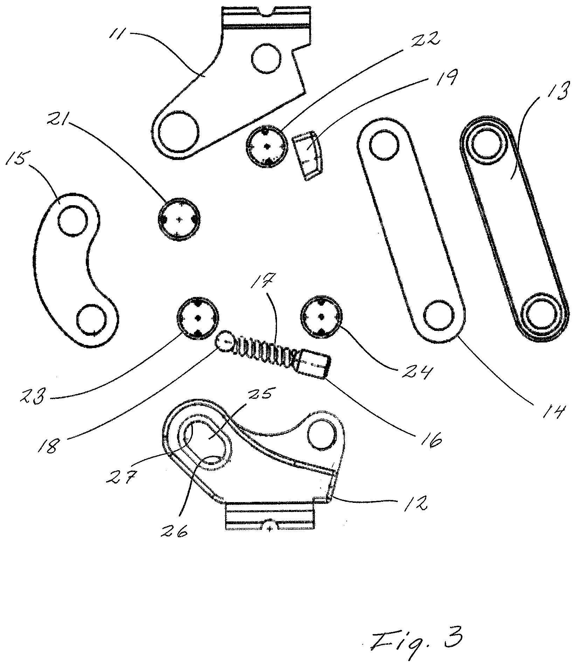

Figure 3 in more detail illustrates the individual components of the invention. Part 11 is the upper part of the joint being attachable to the locking segment of the orthopaedic means of assistance, and part 12 is attachable to the segment of the lower leg. The links being illustrated by part 13, 14 and 15 connect part 11 and part 12 by means of the shaft being illustrated by the parts 21-24. Part 19 is a stop for the links 13 and 14 such that they do not move too long in their movement.

Part 18 is a ball being slidably supporting on a lower end of part 15, and together with a spring 17 and an adjustment screw 16 provides an adjustable adjustment of the force that has to be overcome for the shaft 23 to move downwards/forwards in a in the part 12 formed recess, in which the shaft 23 is movably mounted. In this way, the geometric stability of the knee joint can be adapted to different wearers. The adjustment screw 16 with the spring 17 and the ball 18 is typically inserted and mounted into a boring with internal threads of the lower part 12 of the knee joint and provided for this purpose.

A preferred embodiment of the orthopaedic knee joint thus comprises a first part 11, in which an upper frontal shaft 22 and an upper rear shaft 21 are mounted. In a to the first part 11 articulately connected second part 12, a lower frontal shaft 24 and a lower rear shaft 23 are mounted. A frontal link 13, 14 is rotatably mounted on the frontal shafts 22 and 24, and a rear link 15 is rotatably mounted on the rear shafts 21, 23. A stop 19 is provided on the first part 11 and provided for limiting the rotational motion of the frontal link 13, 14 around the upper frontal shaft 22. The lower rear shaft 23 is slidably mounted in a groove 25 formed in the second part 12, which in relation to the lower frontal shaft 24 extends between a frontal 26 and a distal end 27, wherein the shaft 23 is adjustably pre-tensioned 16, 17, 18 in a direction of the distal end 27.

The pre-tension of the lower rear shaft 23 is generated by means of an against the shaft 23 supporting ball 18 which is actuated by a in the second part 12 mounted spring 17 having a corresponding adjustment screw 16.

The frontal shafts 24 and 22 are on a frontal line L1 and the rear shafts 21, 23 are on a rear line L2, which at extension of the two lines intersects the frontal line L1 at an angle, in an intersectional point MRC in the loaded and straight position of the knee joint is outside the knee joint. The groove 25 has an extension in the second part 12, such that a movement of the shaft 23 in the frontal end 26 of the groove decreases said angle, and moves the intersectional point MRC of the lines further away from the knee joint.

To be more precise, the angle between the frontal line L1 and the rear line L2 will be zero or close to zero when the shaft 23 positions in an end position in the frontal end 26 of the groove 25.

The centre distance between the two shafts 22, 24 is larger than the centre distance between the rear shafts 21, 23, whereby the relation between the length of the frontal link 13, 14 and the length of the rear link 15 results in that the intersectional point MRC is positioned between the frontal shaft 22, 24 in the unloaded, maximum bent position of the knee joint.

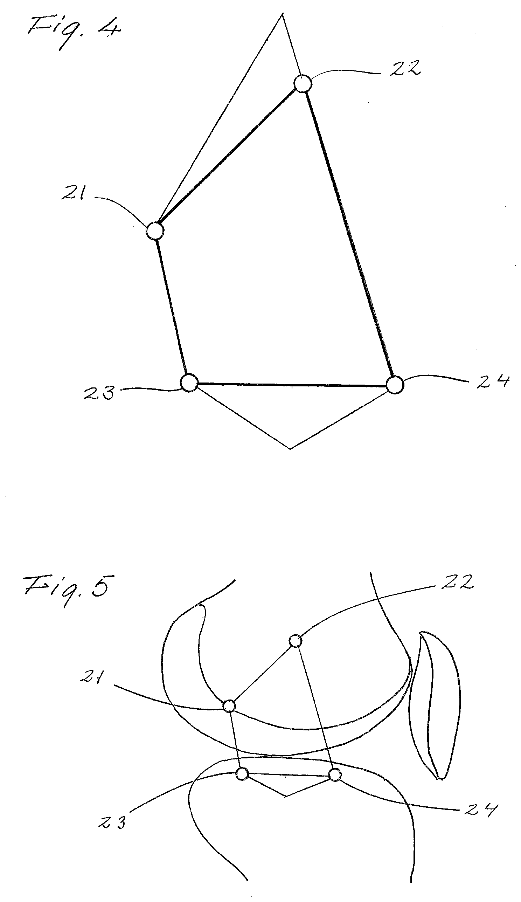

Figure 4 illustrates the theoretical model of the present invention to be able to provide a basic stability by means of a so-called increased instantaneous rotational centre, hereinafter referred to as MRC. This model's function suits the users well that normally require orthosis-support by means of a KAFO, i. e. persons having reduced muscle strength from the hip and below in the extremity. The knee joint 1 has a short rear link between shaft 21 and 23 and a set of longer links between shaft 22 and 24. The intersectional point, i. e. the instantaneous rotational centre MRC between these two links, will be positioned behind the wearer's hip joint (in the extension of the arrows L1-L2 in Figure 2). Since MRC is initially projected behind the hip joint, there is provided a joint being hyper stable throughout the supporting phase, and since it is almost in the same height as the hip joint, there is provided a joint that can be initiated to bending at the end of the supporting phase by means of the wearer generating a small bending torque. The anatomic knee joint is not a monocentric joint but a polycentric joint such that it is more suitable to the movement pattern of this mechanical type of joint together with the anatomic part.

The positioning of the invention on the knee of the wearer must be performed carefully to provide the very best functioning.

Figure 5 illustrates where, in relation to the anatomic knee joint, the invention has to be positioned.

By letting shaft 23 to slide downwards in said groove formed in the part 12 there is provided a slight bending of the knee joint of about 5-10 degrees, as well as link 15 is given the possibility to position parallel to the links 13 and 14. This moves MRC infinitely far away out such that the joint will be stable to that extent that it is not possible to initiate a bending of the knee joint as longs as this geometric locking is present.

Figure 6. Initial Contact: Start of the walking cycle. When the floor reaction force (GRK) is directed anterior if MRC, a stretching torque is generated in the joint which locks the movement of the knee joint, but since GRK is moved closer to shaft 24, an energy saving bending is activated in the knee joint, provided by the bending component, and also providing the joint to be geometrically stable.

Figure 7. Loading Response: In this position during the walking cycle the links of the joint have become totally parallel and the joint is completely stable.

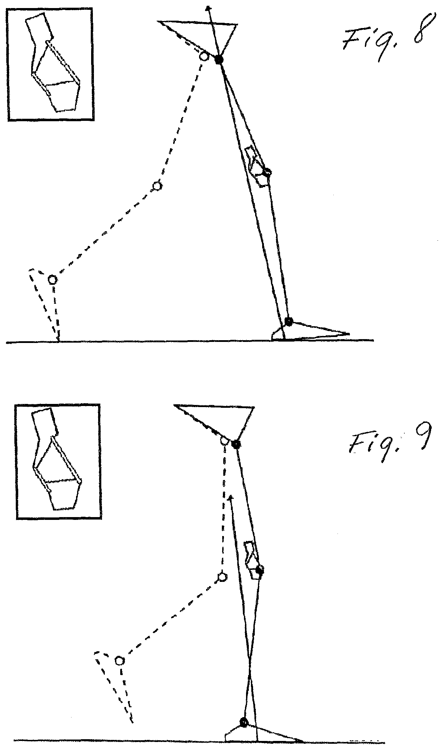

Figure 8. Opposite Toe Off: When the supporting leg supports the total load of the body and the bending component is in maximum position, the knee joint has 7 degrees bending to decrease the vertical movement of the weight of the body. This provides a more energy efficient walking, which is important in particular for users having reduced muscle strength.

Figure 9. Midstance: Mainly the same condition as in previous part of the walking cycle. GRK has such a direction that the knee of the wearer could not be stable without normal muscle strength, unless the geometric stability of the present invention providing this.

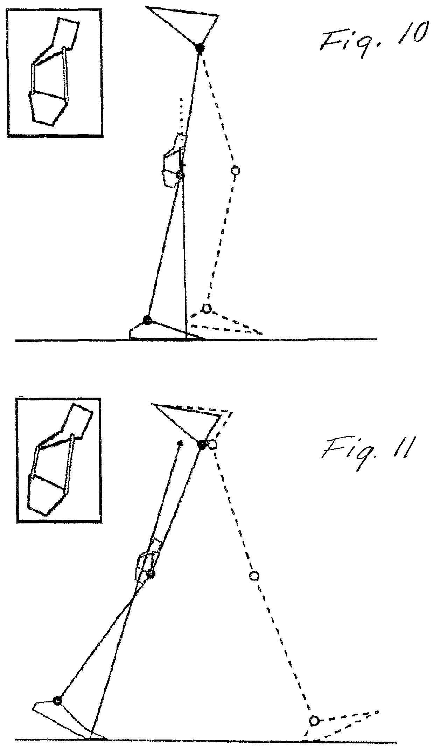

Figure 10. Heel Rise: When the distance at right angles of GRK to shaft 24 is decreased, the bending component will be deactivated gradually to provide the switching to swing phase.

Figure 11. Opposite Initial Contact: Having the bending component deactivated, the joint will be fully straight, and stable as long as GRK is directed anterior to the MRC of the joint. The wearer can in this position also control if the joint should be bent by generating a bending torque in the hip. This manually moves GRK posterior to MRC and in that way a swing phase is initiated. Usually this process occurs at ca 55 % of the length of the walking cycle.

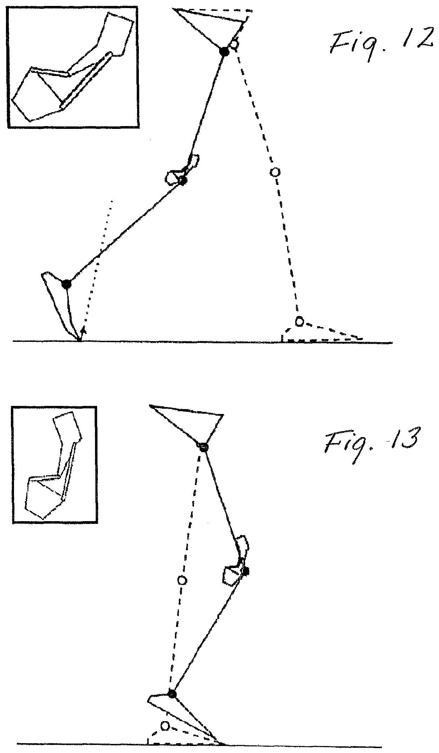

Figure 12. Toe Off: Following Opposite Initial Contact the supporting phase rapidly approach its end and the wearer can either initiate a swing phase by generating a bending torque in the hip joint, or wait until GRK in a natural way moves posterior to MRC, or simply by lifting the leg from the ground. By bending the knee joint, MRC will be moved quickly to the same level as the knee joint 1. Already at 13 degrees bending in the anatomical knee MRC has moved downwards to shaft 22 of the knee joint 1.

Figure 13. Feet Adjacent: In the swing phase the knee joint of the present invention rotates around a MRC positioned on the level of the joint, for angles over 13 degrees, and moves with the anatomic joint centre. The maximum knee bending angle the wearer has in the swing phase is related to the walking speed as well as to the hip strength the wearer has. Lower walking speed gives a lower maximum bending of the knee joint in the swings phase, and lower muscle strength in the hip bending also gives lower bending of the knee joint in the swing phase.

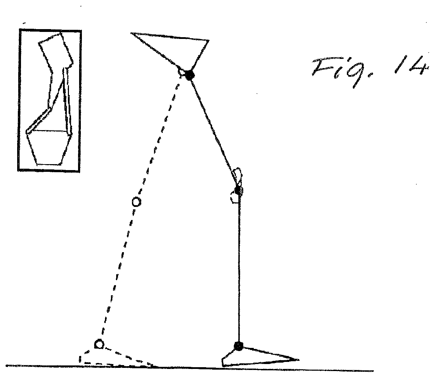

Figure 14. Tibia Vertical: Now the swing phase is approaching its end and a new walking cycle starts with Initial Contact. Between Tibia Vertical and Initial Contact the joint goes against full extension.

This invention discloses a theoretical and practical model for a geometric stand phase-stability incorporated in an orthosis joint provided for supporting a remaining extremity or substitute for a shortened extremity, as well as gives a user the opportunity to an energy efficient walking with a simple and adaptive knee joint orthosis.

As described above there is provided an artificial knee joint which has a first part adapted to be attached to a wearer's first body part or substitute body part and a second part adapted to be attached to a second body part or substitute body part, wherein the first part and the second part can be rotated in relation to each other and are connected to each other via at least four shafts and corresponding links. Characterising for the mechanical knee joint according to the invention is that it is freely movable when the joint is unloaded by the wearer, but in loaded position it is geometrically locked. In particular one of the shafts is slidably mounted in such a way that the geometry of the system of links can be changed such that the joint is locked. Another characterising feature is that the loading condition through the joint influences the stability without the assistance of further control mechanisms, and the degree of stability can be adjusted based on the needs of the wearer.

## Claims

1. Artificial knee joint (1), having a first part (11) adapted to be attached to a wearer's first body part or substitute body part and a second part (12) adapted to be attached to a second body part or substitute body part, which knee joint (1) comprises
a first part (11) in which an upper frontal shaft (22) and an upper rear shaft (21) are mounted,
a to the first part (11) articulately connected second part (12) in which a lower frontal shaft (24) and a lower rear shaft (23) are mounted ,
a frontal link (13; 14) rotatably mounted on the frontal shafts (22, 24),
a rear link (15) rotatably mounted on the rear shafts (21, 23),
a stop (19) provided on the first part (11) and provided for limiting the rotational motion of the frontal link (13,14) around the upper frontal shaft (22),
the lower rear shaft (23) is slidably mounted in a groove (25) formed in the second part (12), which in relation to the lower frontal shaft (24) extends between a frontal (26) and a distal (27) end, **characterised in that** the shaft (23) is adjustably pre-tensioned (16, 17, 18) in a direction of the distal (27) end, the pre-tension of the lower rear shaft (23) is generated by means of an against the shaft (23) supporting ball (18) which is actuated by a in the second part (12) mounted spring (17) having a corresponding adjustment screw (16).

2. Artificial knee joint (1) according to claim 1, **characterised in that** the frontal shaft (24, 22) are on a frontal line (L1), and the rear shaft (21, 23) are on a rear line (L2), which at the extension of the two lines intersects the frontal line (L1) at an angle, in an intersectional point (MRC) in the loaded and straight position of the knee joint (1) is outside the knee joint (1), wherein the groove (25) has an extension in the second part (12), such that a movement of the shaft (23) in a direction directed towards the frontal end (26) of the groove decreases said angle, and moves the intersectional point (MRC) of the lines further away from the knee joint.

3. Artificial knee joint according to claim 2, **characterised in that** the angle between the frontal line (L1) and the rear I(1) ine (L2) is zero or close to zero when the shaft (23) positions in an end position in the frontal end (26) of the groove (25).

4. Artificial knee joint according to claim 2 or 3, **characterised in that** the centre distance between the two shafts (22, 24) is larger than the centre distance between the rear shafts (21, 23), whereby the relation between the length of the frontal link (13, 14) and the length of the rear link (15) results **in that** the intersectional point (MRC) is positioned between the frontal shafts (22, 24) in the unloaded, maximum bent position of the knee joint.

## Patentansprüche

1. Künstliches Kniegelenk (1) mit einem ersten Teil (11), der angepasst ist, um an einem ersten Körperteil oder Ersatzkörperteil eines Trägers befestigt zu werden, und einem zweiten Teil (12), der angepasst ist, um an einem zweiten Körperteil oder Ersatzkörperteil befestigt zu werden, welches Kniegelenk (1) umfasst
einen ersten Teil (11), in dem ein oberer vorderer Schaft (22) und ein oberer hinterer Schaft (21) montiert sind,
einen mit dem ersten Teil (11) gelenkig verbundenen zweiten Teil (12), in dem ein unterer vorderer Schaft (24) und ein unterer hinterer Schaft (23) montiert sind,
eine vordere Verbindung (13; 14), die drehbar an den vorderen Schäften (22, 24) montiert ist,
eine hintere Verbindung (15), die drehbar an den hinteren Schäften (21, 23) montiert ist,
einen Anschlag (19), der an dem ersten Teil (11) vorgesehen ist und zum Begrenzen der Drehbewegung der vorderen Verbindung (13, 14) um den oberen vorderen Schaft (22) vorgesehen ist,
wobei der untere hintere Schaft (23) verschiebbar in einer in dem zweiten Teil (12) ausgebildeten Nut (25) montiert ist, die sich in Bezug auf den unteren vorderen Schaft (24) zwischen einem vorderen (26) und einem distalen (27) Ende erstreckt, **dadurch gekennzeichnet, dass** der Schaft (23) in einer Richtung des distalen (27) Endes einstellbar vorgespannt ist, wobei die Vorspannung des unteren hinteren Schafts (23) mittels einer sich gegen den Schaft (23) abstützenden Kugel (18) erzeugt wird, die durch eine in dem zweiten Teil (12) montierte Feder (17) mit einer entsprechenden Einstellschraube (16) betätigt wird.

2. Künstliches Kniegelenk (1) nach Anspruch 1, **dadurch gekennzeichnet, dass** sich der vordere Schaft (24, 22) auf einer vorderen Linie (L1) befindet und sich der hintere Schaft (21, 23) auf einer hinteren Linie (L2) befindet, die an der Verlängerung der beiden Linien die vordere Linie (L1) in einem Winkel schneidet, in einem Schnittpunkt (MRC) in der belasteten und geraden Position des Kniegelenks (1) ist außerhalb des Kniegelenks (1), wobei die Nut (25) eine Verlängerung in dem zweiten Teil (12) aufweist, so dass eine Bewegung des Schafts (23) in einer zu dem vorderen Ende (26) der Nut gerichteten Richtung den Winkel verringert und den Schnittpunkt (MRC) der Linien weiter von dem Kniegelenk entfernt bewegt.

3. Künstliches Kniegelenk (1) nach Anspruch 2, **dadurch gekennzeichnet, dass** der Winkel zwischen der vorderen Linie (L1) und der hinteren Linie (L2) Null oder nahe Null ist, wenn der Schaft (23) in einer Endposition in dem vorderen Ende (26) der Nut (25) positioniert ist.

4. Künstliches Kniegelenk (1) nach Anspruch 2 oder 3, **dadurch gekennzeichnet, dass** der Achsabstand zwischen den beiden Schäften (22, 24) größer als der Achsabstand zwischen den hinteren Schäften (21, 23) ist, wobei das Verhältnis zwischen der Länge der vorderen Verbindung (13, 14) und der Länge der hinteren Verbindung (15) dazu führt, dass der Schnittpunkt (MRC) zwischen den vorderen Schäften (22, 24) in der unbelasteten, maximal gebeugten Position des Kniegelenks positioniert ist.

## Revendications

1. Articulation artificielle de genou (1), ayant une première partie (11) adaptée pour être fixée à une première partie de corps d'un utilisateur ou partie de corps de substitution et une seconde partie (12) adaptée pour être fixée à une seconde partie de corps ou partie de corps de substitution, laquelle articulation de genou (1) comprend
une première partie (11) dans laquelle un arbre avant supérieur (22) et un arbre arrière supérieur (21) sont montés,
une seconde partie (12) raccordée de manière articulée à la première (11) dans laquelle un arbre avant inférieur (24) et un arbre arrière inférieur (23) sont montés,
une liaison avant (13 ; 14) montée en rotation sur les arbres avant (22, 24),
une liaison arrière (15) montée en rotation sur les arbres arrière (21, 23),
une butée (19) prévue sur la première partie (11) et prévue pour limiter le mouvement de rotation de la liaison avant (13, 14) autour de l'arbre avant supérieur (22),
l'arbre arrière inférieur (23) est monté de manière coulissante dans une rainure (25) formée dans la seconde partie (12), qui s'étend par rapport à l'arbre avant inférieur (24) entre une extrémité avant (26) et distale (27), **caractérisée en ce que** l'arbre (23) est pré-tendu de manière réglable (16, 17, 18) dans une direction de l'extrémité distale (27), la pré-tension de l'arbre arrière inférieur (23) est générée au moyen d'une bille (18) de support contre l'arbre (23) qui est actionnée par un ressort (17) monté dans la seconde partie (12) ayant une vis de réglage correspondante (16).

2. Articulation artificielle de genou (1) selon la revendication 1, **caractérisée en ce que** l'arbre avant (24, 22) est sur une ligne avant (L1), et l'arbre arrière (21, 23) est sur une ligne arrière (L2) qui, au niveau de l'extension des deux lignes, coupe la ligne avant (L1) selon un angle, en un point d'intersection (MRC) dans la position chargée et droite de l'articulation de genou (1) est à l'extérieur de l'articulation de genou (1), dans laquelle la rainure (25) a une extension dans la seconde partie (12), de sorte qu'un mouvement de l'arbre (23) dans une direction dirigée vers l'extrémité avant (26) de la rainure diminue ledit angle, et éloigne davantage le point d'intersection (MRC) des lignes de l'articulation de genou.

3. Articulation artificielle de genou selon la revendication 2, **caractérisée en ce que** l'angle entre la ligne avant (L1) et la ligne arrière (L2) est zéro ou proche de zéro lorsque l'arbre (23) se positionne dans une position d'extrémité dans l'extrémité avant (26) de la rainure (25).

4. Articulation artificielle de genou selon la revendication 2 ou 3, **caractérisée en ce que** la distance de centre à centre entre les deux arbres (22, 24) est supérieure à la distance centre à centre entre les arbres arrière (21, 23), dans laquelle le rapport entre la longueur de la liaison avant (13, 14) et la longueur de la liaison arrière (15) a pour résultat que le point d'intersection (MRC) est positionné entre les arbres avant (22, 24) dans la position pliée maximum, non chargée de l'articulation de genou.
